# EUROPÄISCHE PATENTSCHRIFT

(11) **EP 1 598 021 B1**
(45) Veröffentlichungstag und Bekanntmachung des Hinweises auf die Patenterteilung: **28.06.2006**
(21) Anmeldenummer: 04011687.3
(22) Anmeldetag: 17.05.2004
(51) Int. Cl.: A61B 17/15

(54) **Vorrichtung zum Setzen eines Schnittblocks für eine Resektion der Tibia**
Device for positioning of guide block for tibial resection
Dispositif pour positionner un guide de coupe tibiale

(43) Veröffentlichungstag der Anmeldung: 23.11.2005
(73) Patentinhaber: Zimmer GmbH, 8404 Winterthur (CH)
(72) Erfinder: Overes, Tom, 8404 Winterthur (CH); Fischli, Susanne, 8180 Bülach (CH); Faoro, Francisco, 8002 Zürich (CH); Trachsler, Thomas, 8404 Winterthur (CH)
(74) Vertreter: Manitz, Finsterwald & Partner GbR

(56) Entgegenhaltungen:
- FR-A- 2 703 584
- US-A- 5 628 750
- US-A- 5 681 316
- US-B1- 6 221 035

## Beschreibung

Die Erfindung betrifft eine Vorrichtung zum Setzen eines Schnittblocks gemäß dem Oberbegriff des Anspruchs 1.

Eine derartige Vorrichtung zum Setzen eines Schnittblocks für eine Resektion der Tibia ist grundsätzlich bekannt, beispielsweise aus US 5,628,750. Die Vorrichtung dient insbesondere zur Ausrichtung der Führungsfläche des Schnittblocks an der Tibia, wobei der ausgerichtete Schnittblock mittels Stiften an der Tibia befestigt wird. Anschließend kann auf den Schnittblock eine Sägeblattführung aufgesetzt und mittels eines Sägeblatts die Resektion der Tibia durchgeführt werden. Die bekannte Vorrichtung kann über den Querarm sowohl extramedullär außerhalb des Markraums als auch intramedullär unter Verwendung eines entlang der Tibiaachse gesetzten Marknagels an der Tibia verankert werden.

Bei einer extramedullären Verankerung kann die mit dem Querarm im Wesentlichen rechtwinklig verbundene Säule mittels eines Verlängerungsstabs verlängert und über einen Kreuzschlitten im Bereich des Knöchels an der Tibia abgestützt werden, wobei mittels des entlang der transversalen Achse und der sagittalen Achse verschiebbaren Kreuzschlittens eine Korrektur der Neigung der Führungsfläche des Schnittblocks gegenüber der transversalen bzw. sagittalen Achse erreicht werden kann. Bei einer intramedullären Verankerung kann die Vorrichtung um die Längsachse des Marknagels bzw. die Tibiaachse gedreht werden. Nach korrekter Ausrichtung wird die Vorrichtung, insbesondere deren Querarm, jeweils durch Einschlagen eines oder mehrere Stifte an der Tibia fixiert.

Bei der bekannten Vorrichtung ist der Schnittblock entlang einer Geradführung, die mit dem Querarm verbunden ist und parallel zur Längsachse der Säule verläuft, in der Höhe verstellbar. Der Schnittblock wird dabei über eine Stützfläche einer Verstellmutter, die an der Säule befestigt ist und entlang dieser verstellbar ist, in der jeweiligen Höhe gehalten.

Nachteilig bei derartigen Vorrichtungen zum Setzen eines Schnittblocks mit wenigstens einer Führungsfläche für eine Resektion der Tibia ist jedoch, dass bei extramedullärer Verankerung die Ausrichtung der Vorrichtung lediglich mittels eines zusätzlichen Kreuzschlittens ausgeführt und bei intramedullärer Verankerung die Vorrichtung lediglich um die Längsachse des Marknagels gedreht werden kann.

Darüber hinaus ist auch eine Vorrichtung bekannt, die ein Verschwenken der gesamten Geradführung, entlang der der Schnittblock in der Höhe verstellbar ist, um die sagittale Achse und die transversale Achse ermöglicht. Bei einer derartigen Vorrichtung ist der Querarm zweiteilig ausgeführt, wobei die beiden Bauteile um eine Schwenkachse gegeneinander verschwenkbar sind. Ferner ist zwischen der Geradführung und dem Querarm eine weitere Schwenkachse vorgesehen. Nachteilig hierbei ist jedoch, dass die Vorrichtung einen relativ komplexen Aufbau besitzt und umständlich zu handhaben ist, wobei insbesondere zur Ausführung der Schwenkbewegungen zu betätigende Elemente posterior der Säule und somit nahe der Operationsöffnung angeordnet sind.

Der Erfindung liegt die Aufgabe zugrunde, eine Vorrichtung der eingangs genannten Art zu schaffen, die einen Platz sparenden und zugleich einfachen Aufbau besitzt und möglichst einfach bezüglich der zu resezierenden Tibia ausrichtbar ist, wobei insbesondere eine Korrektur einer Fehlstellung der Beine ermöglicht ist.

Die Lösung dieser Aufgabe erfolgt durch die Merkmale des Anspruchs 1.

Die Erfindung zeichnet sich insbesondere dadurch aus, dass die Trägereinrichtung eine Doppelfunktion erfüllt, da sie einerseits zur Höhenverstellung des Schnittblocks und zugleich zur Korrektur der Neigung der Führungsfläche des Schnittblocks gegen lateral/medial und/oder gegen posterior ausgelegt ist. Die Höhenverstellung des Schnittblocks kann dabei durch Verstellen der Trägereinrichtung an der Säule und die Korrektur der Neigung der Führungsfläche des Schnittblocks durch die Gelenkanordnung der Trägereinrichtung erreicht werden. Hierdurch ergibt sich eine besonders kompakte und somit Platz sparende Ausbildung der erfindungsgemäßen Vorrichtung.

Ein wesentlicher Vorteil der Erfindung besteht darin, dass bei extramedullärer Verankerung die Ausrichtung der Vorrichtung zum Setzen eines Schnittblocks auch ohne Zuhilfenahme eines zusätzlichen Kreuzschlittens ausgeführt, und bei intramedullärer Verankerung auch eine Korrektur der Neigung der Führungsfläche des Schnittblocks gegenüber der transversalen bzw. sagittalen Achse erreicht werden kann.

Erfindungsgemäß ist das den Schnittblock tragende Ende des Trägerarmes posterior und die Gelenkanordnung außerhalb des posterior der Saule gelegenen Halbraumes angeordnet. Hierdurch kann gewährleistet werden, dass zur Ausführung der Korrektur der Neigung der Führungsfläche des Schnittblocks zu betätigende Elemente der Gelenkanordnung und/oder Trägereinrichtung für den Operateur gut zugänglich und von der Operationsöffnung möglichst weit entfernt sind.

Des Weiteren ist erfindungsgemäß vorgesehen, dass die Trägereinrichtung zur Halterung des Schnittblocks einen quer zur Säule angeordneten Trägerarm aufweist, der bevorzugt mit seinen beiden Enden in radialer Richtung über die Säule hervorsteht. Hierdurch ist es insbesondere möglich, die Operation möglichst minimal invasiv durchzuführen. Insbesondere kann der Schnittblock auf das eine Ende des Trägerarms aufgesteckt oder in sonstiger Weise durch diesen gehalten werden. Zur Korrektur der Neigung der Führungsfläche des Schnittblocks werden bevorzugt der Schnittblock und der Trägerarm gemeinsam durch die Gelenkanordnung verstellt.

Vorteilhafte Ausführungsformen der Erfindung sind in den Unteransprüchen, der Beschreibung sowie der Zeichnung angegeben.

Vorzugsweise umfasst die Gelenkanordnung wenigstens zwei insbesondere senkrecht zueinander orientierte Schwenkachsen. Die eine Schwenkachse ist bevorzugt in anterior-/posterior-Richtung orientiert, um eine Korrektur der Neigung der Führungsfläche des Schnittblocks gegen lateral/medial und somit eine Korrektur einer Varus-/Valgusstellung der Beine eines Patienten zu ermöglichen, wohingegen die andere Schwenkachse in medio-lateraler Richtung orientiert ist, um eine Korrektur der Neigung der Führungsfläche des Schnittblocks gegen posterior zu ermöglichen.

Ferner kann erfindungsgemäß der Trägerarm in anterior-/posterior-Richtung verstellbar sein. Hierdurch wird erreicht, dass der durch den Trägerarm gehaltene Schnittblock in einer gegen anterior verschobenen Stellung des Trägerarms auf den Trägerarm aufgesetzt und anschließend durch eine Verschiebung des Trägerarms in posterior-Richtung an die Tibia herangefahren werden kann. Die Korrektur der Neigung der Führungsfläche des Schnittblocks kann in jeder beliebigen Stellung des in anterior-/posterior-Richtung verstellbaren Trägerarms vorgenommen werden. Insbesondere kann der Trägerarm und/oder die Trägereinrichtung mit einer Rastereinrichtung, die lediglich diskrete Stellungen des Trägerarms in anterior-/posterior-Richtung ermöglicht, oder einer Arretiereinrichtung zur Arretierung des Trägerarms in einer bestimmten Stellung in anterior-/posterior-Richtung, versehen sein.

Gemäß einer vorteilhaften Ausführungsform der Erfindung weist die Säule zur Höhenverstellung der Trägereinrichtung eine Geradführung auf, die mit einer Gleiteinrichtung der Trägereinrichtung zusammenwirkt. Mittels der Geradführung und der Gleiteinrichtung kann die Höhenverstellung der Trägereinrichtung an der Säule auf besonders einfache Weise realisiert werden. Die Trägereinrichtung ist hierbei gleitverschieblich an der Säule gehalten.

Bevorzugt ist die Säule zur Ausbildung der Geradführung als Rohr ausgelegt, insbesondere mit einem kreisförmigen oder rechteckigen Innenquerschnitt. Hierdurch kann die Geradführung auf besonders einfache Weise realisiert werden. Alternativ kann die Geradführung aber auch beispielsweise als Kulissenführung oder Schiene an einer Außenseite einer zylinderförmigen Grundform der Säule entlang einer Längsachse der Säule angeordnet sein.

Es ist weiterhin bevorzugt, dass die Gleiteinrichtung wenigstens zwei in Richtung einer Längsachse der Säule voneinander beabstandete, insbesondere als Nutensteine ausgebildete Gleitelemente umfasst. Die beiden Gleitelemente sind zum Zusammenwirken mit der Geradführung der Säule ausgelegt und jeweils gleitverschieblich in der Geradführung der Säule gelagert. Insbesondere kann der Trägerarm zwischen den beiden Gleitelementen angeordnet sein. Hierdurch ergibt sich eine besonders stabile Ausbildung der Trägereinrichtung.

Zur Lagerung in einem insbesondere einen kreisförmigen Innenquerschnitt aufweisenden Rohr sind die Gleitelemente bevorzugt kugelförmig ausgebildet. Zur Lagerung in einem insbesondere einen rechteckigen Innenquerschnitt aufweisenden Rohr sind die Gleitelemente bevorzugt zylinderförmig ausgebildet. Grundsätzlich sind jedoch beliebig ausgebildete Innenquerschnitte des Rohrs und beliebige Formen der Gleitelemente möglich.

Nach einer bevorzugten Ausführungsform der Erfindung ist ein Grundkörper der Trägereinrichtung zur Korrektur der Neigung der Führungsfläche des Schnittblocks gegenüber der sagittalen Achse um eine in medio-lateraler Richtung orientierte erste Schwenkachse relativ zur Säule schwenkbar. Vorzugsweise ist der Trägerarm mit dem Grundkörper um die erste Schwenkachse schwenkbar.

Des Weiteren kann wenigstens ein Gleitelement zur Höhenverstellung der Trägereinrichtung und zugleich zum Schwenken des Grundkörpers der Trägereinrichtung ausgebildet sein. Das Gleitelement kann entlang einer Geradführung der Säule gleitverschieblich gelagert sein und zugleich als Teil eines Drehmechanismus für den Grundkörper wirken, wobei die Translationsbewegung der Trägereinrichtung und die Schwenkbewegung des Grundkörpers einander überlagert sein können.

Nach einer besonders bevorzugten Ausführungsform ist zum Schwenken des Grundkörpers der Abstand zumindest eines Gleitelements zu dem Grundkörper variierbar. Durch die Veränderung des Abstands eines Gleitelements zu dem Grundkörper kann bei zwei in Richtung einer Längsachse der Säule voneinander beabstandeten Gleitelementen der Grundkörper gegenüber der Säule geschwenkt bzw. verkippt werden.

Um eine Schwenk- bzw. Kippbewegung des Grundkörpers gegenüber der Säule zu ermöglichen, wird der Abstand des anderen Gleitelements zu dem Grundkörper bevorzugt konstant gehalten. Alternativ kann der Abstand des anderen Gleitelements zu dem Grundkörper jedoch auch gegensätzlich zu dem Abstand des einen Gleitelements zu dem Grundkörper oder analog zu dem Abstand des einen Gleitelements zu dem Grundkörper, jedoch mit verschiedener Schrittweite verändert werden.

Zur Veränderung des Abstands zwischen dem Gleitelement und dem Grundköper sind das Gleitelement und der Grundkörper bevorzugt über eine Stellverschraubung miteinander verbunden. Wenigstens eines dieser beiden Bauteile, Gleitelement und/oder Grundkörper, kann mit einem Gewinde der Stellverschraubung versehen sein, so dass bei einer Schraubdrehung der Abstand zwischen dem Gleitelement und dem Grundkörper verändert wird.

Vorzugsweise sind die Gleitelemente jeweils über ein insbesondere lang gestrecktes Verbindungselement mit einem Grundkörper verbunden, wobei bevorzugt zumindest ein Verbindungselement mit einem Stellgewinde einer Stellverschraubung versehen ist. Die Stellverschraubung wird dabei durch das Stellgewinde des Verbindungselements und ein Gewinde des Gleitelements und/oder des Grundkörpers gebildet. Die Verbindungselemente sind bevorzugt parallel zu einem Trägerarm der Trägereinrichtung orientiert.

Nach einer besonderen Ausgestaltung der Erfindung ist die Säule zumindest bereichsweise geschlitzt, wobei der Trägerarm insbesondere zwischen zwei Gleitelementen durch den in der Säule ausgebildeten Schlitz hindurchgeführt ist. Hierdurch kann die Vorrichtung zum Setzen eines Schnittblocks besonders Platz sparend und kompakt ausgebildet werden. Alternativ ist jedoch auch eine Vorrichtung denkbar, bei der die Säule nicht geschlitzt ist und der Trägerarm ein- oder zweiseitig um die Säule herum geführt ist.

Zur Drehsicherung des Trägerarms gegen eine Drehung um eine Längsachse der Säule steht bevorzugt wenigstens ein Sicherungselement der Trägereinrichtung mit dem Schlitz in Eingriff. Das von der Trägereinrichtung abgewandte Ende des Sicherungselements kann innerhalb der Säule angeordnet sein oder aber auf der der Trägereinrichtung gegenüberliegenden Seite der Säule aus dieser hervorragen. Das Sicherungselement kann an einem Grundkörper der Trägereinrichtung und/oder an einem Gleitelement der Trägereinrichtung angebracht sein.

Nach einer weiteren bevorzugten Ausführungsform der Erfindung ist ein Halterungselement der Trägereinrichtung zur Korrektur der Neigung der Führungsfläche des Schnittblocks gegenüber der transversalen Achse um eine in anterior-/posterior-Richtung orientierte zweite Schwenkachse schwenkbar. Vorzugsweise ist der Trägerarm mit dem Halterungselement um die zweite Schwenkachse schwenkbar. Insbesondere ist das Halterungselement als Mitnehmer für den Trägerarm ausgebildet. Zum Schwenken des Halterungselements ist bevorzugt ein in medio-lateraler Richtung verstellbarer Gewindestab vorgesehen, in den das Halterungselement eingreift.

Die Neigung der Führungsfläche des Schnittblocks gegen lateral/medial und/oder gegen posterior ist besonders einfach erkennbar, wenn der Winkel einer Schwenkbewegung um eine erste und/oder zweite Achse jeweils auf einer Skala ablesbar ist. Insbesondere können hierdurch die bei der Operationsplanung mittels Röntgenbildern bestimmten Korrekturwinkel auf einfache Weise und zuverlässig eingestellt werden.

Die Erfindung wird im Folgenden beispielhaft unter Bezugnahme auf die Zeichnung beschrieben. Es zeigen:
- Fig. 1: eine Seitenansicht einer erfindungsgemäßen Vorrichtung zum Setzen eines Schnittblocks,
- Fig. 2a, b: Ansichten der Vorrichtung von Fig. 1 von posterior bzw. anterior,
- Fig. 3a, b: Ansichten der Vorrichtung von Fig. 1 von proximal bzw. distal,
- Fig. 4: eine längsgeschnittene Seitenansicht der Vorrichtung von Fig. 1 entlang der Linie A-A in Fig. 2a, und
- Fig. 5: eine längsgeschnittene Seitenansicht einer weiteren Ausführungsform der erfindungsgemäßen Vorrichtung.

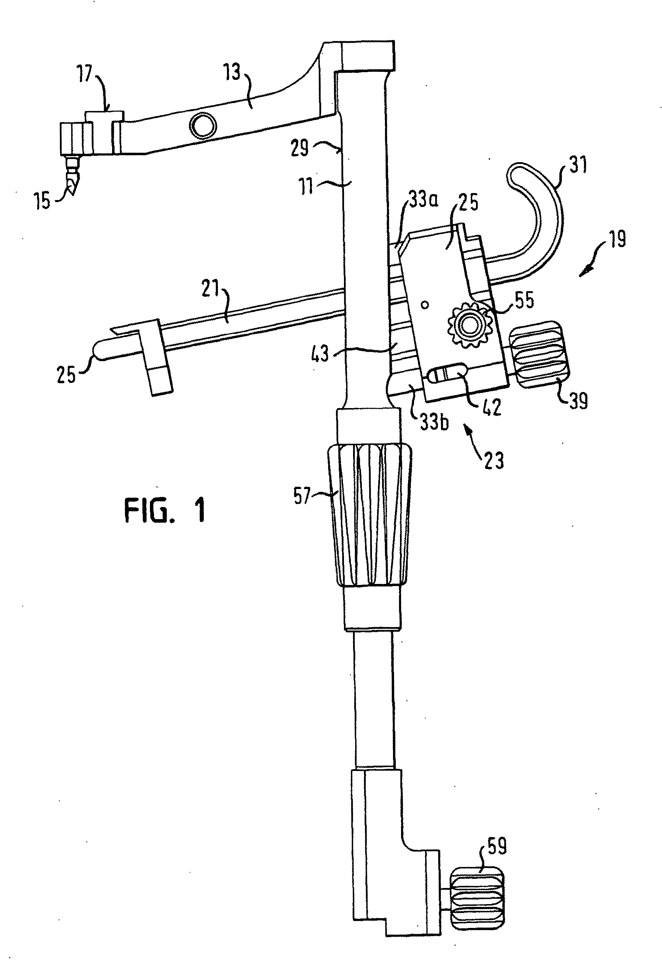

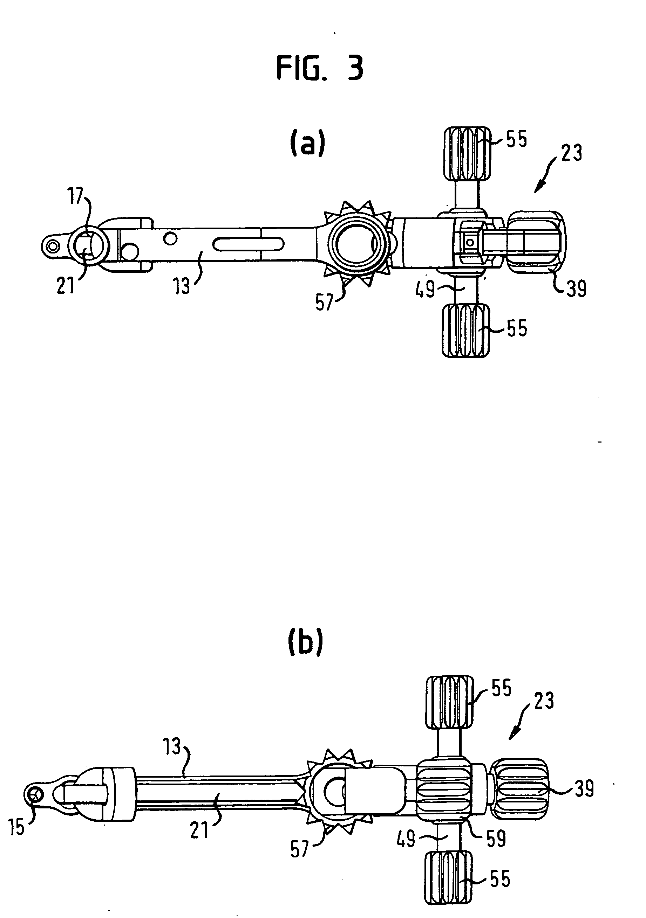

Die Fig. 1 bis 3 zeigen eine erfindungsgemäße Vorrichtung zum Setzen eines nicht dargestellten Schnittblocks mit Führungsflächen für eine Resektion einer nicht dargestellten Tibia aus verschiedenen Richtungen. Die Vorrichtung umfasst eine bezüglich der Tibia ausrichtbare Säule 11 und einen mit der Säule 11 im Wesentlichen rechtwinklig verbundenen Querarm 13, der extramedullär oder intramedullär an der natürlichen Tibiaplattform verankerbar ist. Bei extramedullärer Verankerung wird ein vom Querarm 13 nach distal vorstehender Stift 15 gesetzt. Bei intramedullärer Verankerung wird zusätzlich ein nicht dargestellter Marknagel gesetzt, der entlang einer in dem Querarm 13 ausgebildeten Führungsbohrung 17 in die Tibia eingeschlagen wird.

Die erfindungsgemäße Setzvorrichtung umfasst ferner eine Trägereinrichtung 19, die an der Säule 11 entlang einer Längsachse der Säule 11 in der Höhe verstellbar ist. Die Trägereinrichtung 19 weist einen quer zur Säule 11 angeordneten Trägerarm 21 zur Halterung des Schnittblocks und eine Gelenkanordnung 23 auf, die das Einstellen unterschiedlicher Neigungen der Führungsflächen des durch den Trägerarm 21 gehaltenen Schnittblocks gegen lateral/medial und gegen posterior erlaubt. Das den Schnittblock tragende Ende 25 des Trägerarms 21 ist posterior und die Gelenkanordnung 23 anterior der Säule 11 angeordnet.

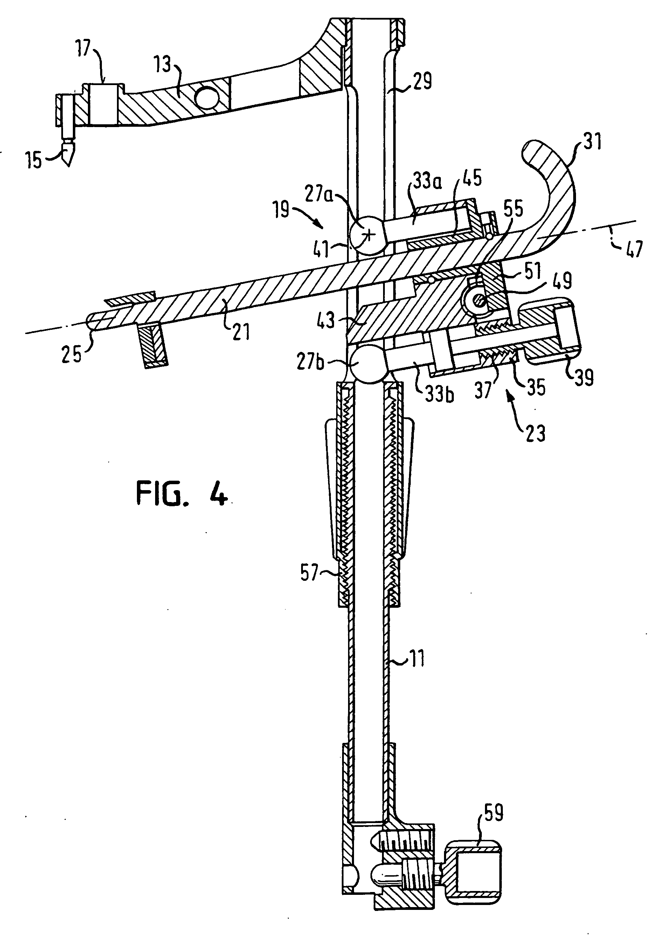

Gemäß Fig. 4 ist die Säule 11 als Rohr mit einem kreisförmigen Innenquerschnitt ausgebildet. Das Rohr 11 bildet eine in Längsrichtung der Säule 11 orientierte Geradführung, in die zwei in Längsrichtung der Säule 11 voneinander beabstandete, kugelförmige Nutensteine 27 der Gelenkanordnung 23 gleitverschieblich eingesetzt sind. Die beiden Nutensteine 27 bilden eine Gleiteinrichtung der Gelenkanordnung 23, mittels der die Trägereinrichtung 19, d.h. die Gelenkanordnung 23 und der Trägerarm 21, in der Höhe verstellbar sind.

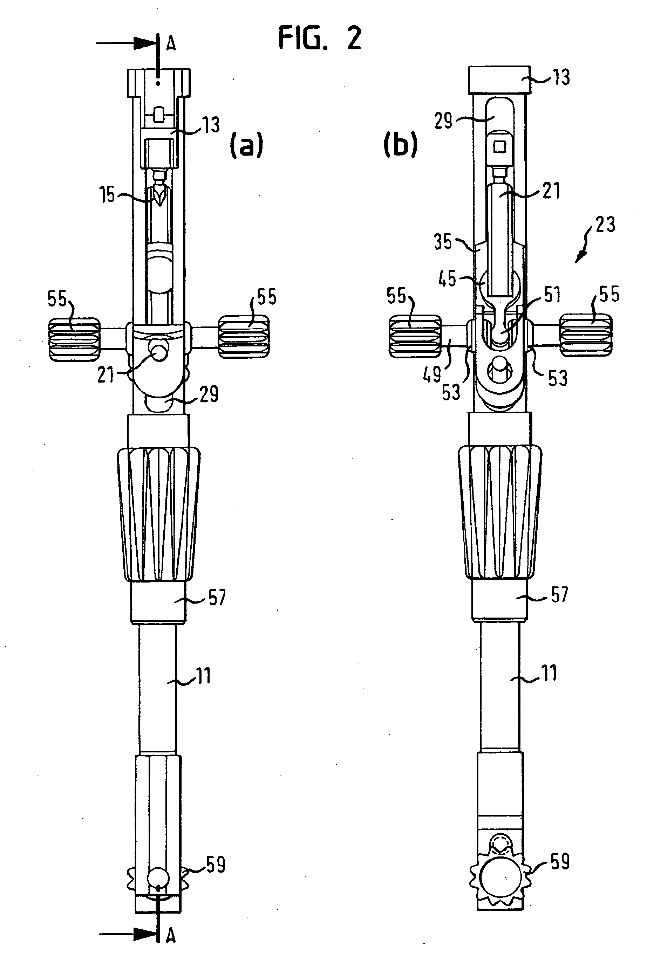

Der Trägerarm 21, der zusätzlich in anterior-/posterior-Richtung verstellbar ist, ist zwischen den beiden Nutensteinen 27 durch einen in der Säule 11 ausgebildeten, die Säule 11 von anterior nach posterior durchdringenden und sich in Längsrichtung der Säule 11 erstreckenden Schlitz 29 geführt, wie insbesondere in Fig. 2 zu erkennen ist. Der Schlitz 29 durchdringt dabei sowohl die nach anterior als auch die nach posterior gewandte Wand der rohrförmigen Säule 11. Das dem den Schnittblock tragenden Ende 25 entgegengesetzte Ende 31 des Trägerarms 21 ist gekrümmt und in Form einer Eingriffsmulde ausgebildet, in die der Operateur auf einfache Weise mit einem Finger eingreifen kann, um den Trägerarm 21 in anterior-/posterior-Richtung zu verstellen.

Die Nutensteine 27 sind über jeweils ein lang gestrecktes Verbindungselement 33 mit einem Grundkörper 35 der Gelenkanordnung 23 verbunden, wobei die Verbindungselemente 33 durch den Schlitz 29 geführt sind. Die beiden Verbindungselemente 33 sind fest mit dem jeweiligen Nutenstein 27 verbunden. Das dem oberhalb des Trägerarms 21 angeordneten oberen Nutenstein 27a zugeordnete obere Verbindungselement 33a ist weiterhin fest mit dem Grundkörper 35 verbunden.

Das dem unterhalb des Trägerarms 21 angeordneten unteren Nutenstein 27b zugeordnete untere Verbindungselement 33b ist hingegen über eine Stellverschraubung 37 mit dem Grundkörper 35 verbunden. Zur Ausbildung der Stellverschraubung 37 ist das Verbindungselement 33b mit einem Stellgewinde in Form eines Außengewindes und der Grundkörper 35 mit einem entsprechenden Innengewinde versehen. Mittels der Stellverschraubung 37 kann der Abstand zwischen dem unteren Nutenstein 27b und dem Grundkörper 35 variiert werden. Zum Verstellen der Stellverschraubung 37 ist ein Drehknopf 39, der in Fig. 2b der Übersichtlichkeit halber nicht dargestellt ist, vorgesehen.

Der Grundkörper 35 ist zur Korrektur der Neigung der Führungsflächen des Schnittblocks gegenüber der sagittalen Achse um eine in medio-lateraler Richtung orientierte, in Fig. 4 senkrecht zur Zeichenebene verlaufende erste Schwenkachse 41 relativ zur Säule 11 schwenkbar. Eine Schwenkbewegung um die erste Schwenkachse 41 kann durch Drehen des Drehknopfs 39, d.h. durch Verändern des Abstands zwischen dem unteren Nutenstein 27b und dem Grundkörper 35, bewirkt werden, da der Abstand zwischen dem oberen Nutenstein 27a und dem Grundkörper 35 fest ist. Der untere Nutenstein 27b ist somit sowohl zur Höhenverstellung der Trägereinrichtung 19 als auch zum Schwenken des Grundkörpers 35 ausgebildet. Der Winkel der Schwenkbewegung des Grundkörpers 35 um die erste Schwenkachse 41 kann auf einer Skala 42, die in Fig. 1 dargestellt ist, abgelesen werden.

Zur Drehsicherung des Trägerarms 21 bzw. der Trägereinrichtung 19 um die Längsachse der Säule 11 ist ein Sicherungselement 43 vorgesehen, dessen zumindest eine Abmessung derart auf die Breite des Schlitzes 29 abgestimmt ist, dass das Sicherungselement 43 zumindest annähernd spielfrei mit dem Schlitz 29 der Säule 11 in Eingriff steht. Das Sicherungselement 43 ist an dem Grundkörper 35 angebracht und zwischen dem Trägerarm 21 und dem unteren Nutenstein 27b angeordnet.

Zur Korrektur der Neigung des Trägerarms 21 gegenüber der transversalen Achse ist ein Halterungselement 45 der Trägereinrichtung 19 um eine in anterior-/posterior-Richtung orientierte zweite Schwenkachse 47 schwenkbar, die senkrecht zu der ersten Schwenkachse 41 verläuft. Der Trägerarm 21 besitzt in einer senkrecht zur zweiten Schwenkachse 47 verlaufenden Ebene einen rechteckigen Außenquerschnitt, der auf einen rechteckigen Innenquerschnitt des Halterungselements 45 angepasst ist, wie in Fig. 2b zu erkennen ist. Das Halterungselement 45 ist gewissermaßen als Mitnehmer für den Trägerarm 21 ausgebildet.

Zum Schwenken des Halterungselements 45 ist ein in medio-lateraler Richtung verstellbarer Gewindestab 49 vorgesehen, in den ein insbesondere in Fig. 2b dargestellter Klöppel 51 des Halterungselements 45 eingreift. Der Klöppel 51 ist zwischen einem ersten und einem zweiten Außengewindeabschnitt 53 des Gewindestabs 49 angeordnet. Die Außengewindeabschnitte 53 bilden mit entsprechenden, nicht dargestellten Innengewindeabschnitten des Grundkörpers 35 eine weitere Stellverschraubung. Der zwischen den beiden Außengewindeabschnitten 53 ausgebildete Bereich des Gewindestabs 49, in den der Klöppel 51 eingreift, weist kein Gewinde auf.

An den beiden Enden des Gewindestabs 49 ist jeweils ein Drehknopf 55 angebracht. Durch Drehen eines der beiden Drehknöpfe 55 kann der Gewindestab 49 in medio-lateraler Richtung verstellt werden. Dabei werden auch die beiden Außengewindeabschnitte 53 und der zwischen den beiden Außengewindeabschnitten 53 ausgebildete Bereich, in den der Klöppel 51 eingreift, und der Klöppel 51 entsprechend verschoben. Mit der Verschiebung des Klöppels 51 in medio-lateraler Richtung wird das Halterungselement 47, das in dem Grundkörper drehbeweglich gelagert ist, und somit der Trägerarm 21 um die zweite Schwenkachse 47 geschwenkt.

Die Verstellbewegung des Gewindestabs 49 in medio-lateraler Richtung ist beidseitig begrenzt, so dass der Trägerarm 21 lediglich bis zu einer gewissen Winkelstellung im und entgegen den Uhrzeigersinn um die zweite Schwenkachse 47 geschwenkt werden kann. Insbesondere kann hierdurch ein Anschlagen eines quer gestellten Trägerarms 21 an den Begrenzungen des Schlitzes 29 verhindert werden.

Gegebenenfalls vorhandenes Spiel zwischen dem Halterungselement 47 und dem Trägerarm 21 kann durch beispielsweise ein federbelastetes Druckelement des einen Elements 21, 47, das in eine prismatische Gegenfläche des anderen Elements 47, 21 hineingedrückt wird, oder beliebige sonstige Spiel verringernde Maßnahmen beseitigt werden.

Zur Höhenverstellung der Trägereinrichtung 19 ist eine Verstellmutter 57 vorgesehen, die an der Säule 11 befestigt und entlang der Längsrichtung der Säule 11 mittels eines Innengewindes, das in ein Außengewinde der Säule 11 eingreift, verstellbar ist. Die Trägereinrichtung 19 wird durch die Verstellmutter 57 auf der jeweiligen durch die Verstellmutter 57 eingenommen Höhe gehalten. Das Außengewinde der Säule 11 erstreckt sich auch auf den den Schlitz 29 aufweisenden Bereich der Säule 11.

Zur zusätzlichen Abstützung der erfindungsgemäßen Vorrichtung bei extramedullärer Verankerung kann am unteren Ende der Säule 11 ein nicht dargestellter Verlängerungsstab eingeführt werden, der über einen nicht dargestellten Kreuzschlitten im Bereich des Knöchels an der Tibia abgestützt wird. Zur Befestigung des Verlängerungsstabs ist eine an der Säule 11 angebrachte Klemmschraube 59 vorgesehen.

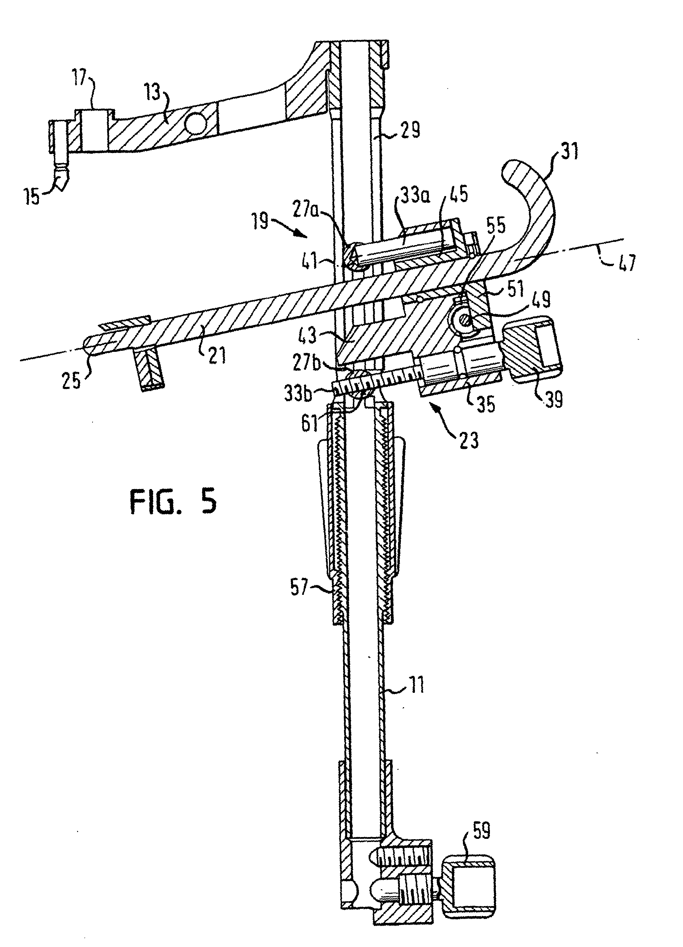

Fig. 5 zeigt ein weiteres Ausführungsbeispiel einer erfindungsgemäßen Setzvorrichtung, wobei jeweils gleiche oder entsprechende Teile mit denselben Bezugszeichen bezeichnet sind, so dass auf eine erneute Vorstellung verzichtet wird und lediglich die Abweichungen der in diesen Figuren dargestellten Ausführungsform gegenüber der ersten Ausführungsform erläutert werden.

Im Gegensatz zu dem Ausführungsbeispiel gemäß den Fig. 1 bis 4 sind bei der in Fig. 5 gezeigten Vorrichtung das untere Verbindungselement 33b und der Grundkörper 35 nicht mittels einer Stellverschraubung, sondern fest miteinander verbunden. Um eine Veränderung des Abstands des unteren Nutensteins 27b zu dem Grundkörper 35 zu ermöglichen, sind bei dem weiteren Ausführungsbeispiel gemäß Fig. 5 der untere Nutenstein 27b und das untere Verbindungselement 33b mittels einer Stellverschraubung 61 miteinander verbunden. Eine Schwenkbewegung des Grundkörpers 35 um die erste Schwenkachse 41 kann in Analogie zu dem in den Fig. 1 bis 4 dargestellten Ausführungsbeispiel mittels des Drehknopfs 39 bewirkt werden.

Die anhand der beiden Ausführungsbeispiele erläuterte erfindungsgemäße Vorrichtung zum Setzen eines Schnittblocks umfasst eine kompakte Gelenkanordnung, die einerseits gemeinsam mit dem Trägerarm an der Säule der Vorrichtung in der Höhe verstellbar ist und andererseits eine Korrektur der Neigung der Führungsflächen eines Schnittblocks gegen lateral/medial und posterior erlaubt. Die Gelenkanordnung ist trotz der Doppelfunktionalität äußerst Platz sparend ausgebildet und kann durch den Operateur auf besonders einfache Weise bedient werden.

### Bezugszeichenliste

- 11: Säule
- 13: Querarm
- 15: Stift
- 17: Führungsbohrung
- 19: Trägereinrichtung
- 21: Trägerarm
- 23: Gelenkanordnung
- 25: Ende des Trägerarms
- 27: Nutenstein
- 29: Schlitz
- 31: Ende des Trägerarms
- 33: Verbindungselement
- 35: Grundkörper
- 37: Stellverschraubung
- 39: Drehknopf
- 41: Schwenkachse
- 42: Skala
- 43: Sicherungselement
- 45: Halterungselement
- 47: Schwenkachse
- 49: Gewindestab
- 51: Klöppel
- 53: Gewindeabschnitt
- 55: Drehknopf
- 57: Verstellmutter
- 59: Klemmschraube
- 61: Stellverschraubung

## Patentansprüche

1. Vorrichtung zum Setzen eines Schnittblocks mit wenigstens einer Führungsfläche für eine Resektion der Tibia, die sowohl extramedullär als auch intramedullär an der Tibia verankerbar ist, mit einer bezüglich der Tibia ausrichtbaren Säule (11), die mit einem zur Verankerung an der natürlichen Tibiaplattform vorgesehenen Querarm (13) verbunden ist, wobei der Schnittblock relativ zum Querarm (13) in der Höhe verstellbar ist, wobei
für den Schnittblock eine an der Säule (11) in der Höhe verstellbare Trägereinrichtung (19) vorgesehen ist, die eine Gelenkanordnung (23) aufweist, durch die unterschiedliche Neigungen der Führungsfläche des Schnittblocks gegen lateral/medial und/oder gegen posterior einstellbar sind,
wobei die Trägereinrichtung (19) zur Halterung des Schnittblocks einen quer zur Säule (11) angeordneten Trägerarm (21) aufweist, **dadurch gekennzeichnet, daß**
ein den Schnittblock tragendes Ende (25) des Trägerarmes (21) posterior und die Gelenkanordnung (23) außerhalb des posterior der Säule (11) gelegenen Halbraumes angeordnet ist.

2. Vorrichtung nach Anspruch 1,
**dadurch gekennzeichnet,**
**dass** die Gelenkanordnung (23) wenigstens zwei insbesondere senkrecht zueinander orientierte Schwenkachsen (41, 47) umfasst.

3. Vorrichtung nach Anspruch 1 oder 2,
**dadurch ggekennzeichnet,**
**dass** der Trägerarm (21) in anterior-/posterior-Richtung verstellbar ist.

4. Vorrichtung nach einem der vorhergehenden Ansprüche,
**dadurch gekennzeichnet,**
**dass** die Säule (11) zur Höhenverstellung der Trägereinrichtung (19) eine Geradführung aufweist, die mit einer Gleiteinrichtung (27) der Trägereinrichtung (19) zusammenwirkt.

5. Vorrichtung nach Anspruch 4,
**dadurch gekennzeichnet,**
**dass** die Säule zur Ausbildung der Geradführung als Rohr (11) ausgelegt ist, insbesondere mit einem kreisförmigen oder rechteckigen Innenquerschnitt.

6. Vorrichtung nach Anspruch 4 oder 5,
**dadurch gekennzeichnet,**
**dass** die Gleiteinrichtung wenigstens zwei in Richtung einer Längsachse der Säule (11) voneinander beabstandete, insbesondere als Nutensteine ausgebildete Gleitelemente (27) umfasst.

7. Vorrichtung nach Anspruch 6,
**dadurch gekennzeichnet,**
**dass** die Gleitelemente (27) zur Lagerung in einem insbesondere einen kreisförmigen Innenquerschnitt aufweisenden Rohr (11) kugelförmig ausgebildet sind.

8. Vorrichtung nach Anspruch 6,
**dadurch gekennzeichnet,**
**dass** die Gleitelemente (27) zur Lagerung in einem insbesondere einen rechteckigen Innenquerschnitt aufweisenden Rohr (11) zylinderförmig ausgebildet sind.

9. Vorrichtung nach einem der vorhergehenden Ansprüche,
**dadurch gekennzeichnet,**
**dass** ein Grundkörper (35) der Trägereinrichtung (19) zur Korrektur der Neigung der Führungsfläche des Schnittblocks gegenüber der sagittalen Achse um eine in medio-lateraler Richtung orientierte erste Schwenkachse (41) relativ zur Säule (11) schwenkbar ist.

10. Vorrichtung nach Anspruch 9,
**dadurch gekennzeichnet,**
**dass** wenigstens ein Gleitelement (27b) zur Höhenverstellung der Trägereinrichtung (19) und zugleich zum Schwenken des Grundkörpers (35) der Trägereinrichtung (19) ausgebildet ist.

11. Vorrichtung nach Anspruch 9 oder 10,
**dadurch gekennzeichnet,**
**dass** zum Schwenken des Grundkörpers (35) der Abstand zumindest eines Gleitelements (27b) zu dem Grundkörper (35) variierbar ist.

12. Vorrichtung nach. Anspruch 11,
**dadurch gekennzeichnet ,**
**dass** das Gleitelement (27b) und der Grundkörper (35) über eine Stellverschraubung (37) miteinander verbunden sind.

13. Vorrichtung nach einem der Ansprüche 6 bis 12,
**dadurch gekennzeichnet,**
**dass** die Gleitelemente (27) jeweils über ein insbesondere lang gestrecktes Verbindungselement (33) mit einem Grundkörper (35) verbunden sind, wobei bevorzugt zumindest ein Verbindungselement (33b) mit einem Stellgewinde einer Stellverschraubung (37) versehen ist.

14. Vorrichtung nach einem der vorhergehenden Ansprüche,
**dadurch gekennzeichnet,**
**dass** die Säule (11) zumindest bereichsweise geschlitzt ist, wobei der Trägerarm (21) insbesondere zwischen zwei Gleitelementen (27) durch den in der Säule (11) ausgebildeten Schlitz (29) hindurchgeführt ist.

15. Vorrichtung nach Anspruch 14,
**dadurch ggekennzeichnet,**
**dass** wenigstens ein Sicherungselement (43) der Trägereinrichtung (19) zur Drehsicherung des Trägerarms (21) gegen eine Drehung um eine Längsachse der Säule (11) mit dem Schlitz (29) in Eingriff steht.

16. Vorrichtung nach Anspruch 15,
**dadurch gekennzeichnet,**
**dass** das Sicherungselement (43) an einem Grundkörper (35) der Trägereinrichtung (19) angebracht ist.

17. Vorrichtung nach Anspruch 15 oder 16,
**dadurch gekennzeichnet,**
**dass** das Sicherungselement (43) an einem Gleitelement (27) der Trägereinrichtung (19) angebracht ist.

18. Vorrichtung nach einem der vorhergehenden Ansprüche,
**dadurch gekennzeichnet,**
**dass** ein Halterungselement (45) der Trägereinrichtung (19) zur Korrektur der Neigung der Führungsfläche des Schnittblocks gegenüber der transversalen Achse um eine in anterior-/posterior-Richtung orientierte zweite Schwenkachse (47) schwenkbar ist.

19. Vorrichtung nach Anspruch 18,
**dadurch gekennzeichnet,**
**dass** das Halterungselement (45) als Mitnehmer für den Trägerarm (21) ausgebildet ist.

20. Vorrichtung nach Anspruch 18 oder 19,
**dadurch gekennzeichnet,**
**dass** zum Schwenken des Halterungselements (45) ein in medio-lateraler Richtung verstellbarer Gewindestab (49) vorgesehen ist, in den das Halterungselement (45) eingreift.

21. Vorrichtung nach einem der vorhergehenden Ansprüche,
**dadurch gekennzeichnet,**
**dass** der Winkel einer Schwenkbewegung um eine erste und/oder zweite Achse (41, 47) jeweils auf einer Skala (42) ablesbar ist.

## Claims

1. An apparatus for setting a cutting block having at least one guide surface for a resection of the tibia, the apparatus being capable of being anchored to the tibia in both an extramedullary and an intramedullary manner, and comprising a column (11) which can be aligned with respect to the tibia and which is connected to a transverse arm (13) provided for the anchoring at the natural tibia platform, with the cutting block being vertically adjustable relative to the transverse arm (13), wherein a carrier device (19) which is vertically adjustable at the column (11) is provided for the cutting block and has a joint arrangement (23) by which different inclinations of the guide surface of the cutting block can be set toward lateral /medial and/or toward posterior,
wherein the carrier device (19) has a carrier arm (21) arranged transversely to the column (11) for the holding of the cutting block
**characterized in that**
an end (25) of the carrier arm (21) carrying the cutting block is arranged to posterior and the joint arrangement (23) is arranged outside the hollow space disposed to posterior of the column (11).

2. An apparatus in accordance with claim 1, **characterized in that** the joint arrangement (23) includes at least two pivot axes (41, 47) in particular oriented perpendicular to one another.

3. An apparatus in accordance with claim 1 or claim 2, **characterized in that** the carrier arm (21) is adjustable in the anterior/posterior direction.

4. An apparatus in accordance with any one of the preceding claims, **characterized in that** the column (11) has a straight-line guide for the vertical adjustment of the carrier device (19) which cooperates with a slide device (27) of the carrier device (19).

5. An apparatus in accordance with claim 4, **characterized in that** the column is configured for the making of the straight-line guide as a tube (11), in particular having a circular or a rectangular inner cross-section.

6. An apparatus in accordance with claim 4 or claim 5, **characterized in that** the slide device includes at least two slide elements (27) which are spaced apart from one another in the direction of a longitudinal axis of the column (11) and are in particular made as groove blocks.

7. An apparatus in accordance with claim 6, **characterized in that** the slide elements (27) are spherical for the support in a tube (11) in particular having a circular internal cross-section.

8. An apparatus in accordance with claim 6, **characterized in that** the slide elements (27) are cylindrical for the support in a tube (11) in particular having a rectangular internal cross-section.

9. An apparatus in accordance with any one of the preceding claims, **characterized in that** a base body (35) of the carrier device (19) is pivotable relative to the column (11) about a first pivot axis (41) oriented in the medio-lateral direction for the correction of the inclination of the guide surface of the cutting block with respect to the sagittal axis.

10. An apparatus in accordance with claim 9, **characterized in that** at least one slide element (27b) is made for the vertical adjustment of the carrier device (19) and simultaneously for the pivoting of the base body (35) of the carrier device (19).

11. An apparatus in accordance with claim 9 or claim 10, **characterized in that** the spacing of at least one slide element (27b) from the base body (35) can be varied for the pivoting of the base body (35).

12. An apparatus in accordance with claim 11, **characterized in that** the slide element (27b) and the base body (35) are connected to one another via a regulating screw connection (37).

13. An apparatus in accordance with any one of the claims 6 to 12, **characterized in that** the slide elements (27) are each connected to a base body (35) via a connection element (33), in particular via an elongated connection element, with preferably at least one connection element (33b) being provided with a regulating thread of a regulating screw connection (37).

14. An apparatus in accordance with any one of the preceding claims, **characterized in that** the column (11) is slotted at least regionally, with the carrier arm (21) in particular being guided between two slide elements (27) through the slot (29) formed in the column (11).

15. An apparatus in accordance with claim 14, **characterized in that** at least one securing element (43) of the carrier device (19) is in engagement with the slot (29) for the rotational security of the carrier arm (21) with respect to a rotation about a longitudinal axis of the column (11).

16. A method in accordance with claim 15, **characterized in that** the securing element (43) is attached to a base body (35) of the carrier device (19).

17. An apparatus in accordance with claim 15 or claim 16, **characterized in that** the securing element (43) is attached to a slide element (27) of the carrier device (19).

18. An apparatus in accordance with any one of the preceding claims, **characterized in that** a holder element (45) of the carrier device (19) is pivotable about a second pivot axis (47) oriented in the anterior/posterior direction for the correction of the inclination of the guide surface of the cutting block with respect to the transverse axis.

19. An apparatus in accordance with claim 18, **characterized in that** the holder element (45) is made as a driver for the carrier arm (21).

20. An apparatus in accordance with claim 18 or claim 19, **characterized in that**, to pivot the holder element (45), a threaded rod (49) adjustable in the medio-lateral direction is provided into which the holder element (45) engages.

21. An apparatus in accordance with any one of the preceding claims, **characterized in that** the angle of a pivot movement about a first and/or second axis (41, 47) can be read off at a scale (42).

## Revendications

1. Dispositif pour poser un bloc de coupe avec au moins une surface de guidage pour une résection du tibia, susceptible d'être ancré sur le tibia aussi bien en situation extramédullaire qu'en situation intramédullaire, comprenant une colonne (11) susceptible d'être alignée par rapport au tibia et qui est reliée à un bras transversal (13) prévu pour l'ancrage sur la plate-forme tibiale naturelle, ledit bloc de coupe étant réglable en hauteur par rapport au bras transversal (13), dans lequel il est prévu pour le bloc de coupe un dispositif porteur (19) réglable en hauteur sur la colonne (11), qui comporte un agencement articulé (23) au moyen duquel il est possible de régler des inclinaisons différentes de la surface de guidage du bloc de coupe en direction latérale/médiale et/ou en direction postérieure, le dispositif porteur (19) comportant un bras porteur (21) agencé transversalement à la colonne (11) pour le support du bloc de coupe, **caractérisé en ce qu'**une extrémité (25) du bras porteur (21) qui porte le bloc de coupe est agencée en situation postérieure, et l'agencement articulé (23) est agencé à l'extérieur du demi-espace situé en direction postérieure de la colonne (11).

2. Dispositif selon la revendication 1, **caractérisé en ce que** l'agencement articulé (23) comprend au moins deux axes de pivotement (41, 47) orientés en particulier perpendiculairement l'un à l'autre.

3. Dispositif selon la revendication 1 ou 2, **caractérisé en ce que** le bras porteur (21) est réglable en direction antérieure/postérieure.

4. Dispositif selon l'une des revendications précédentes, **caractérisé en ce que** la colonne comprend un guidage linéaire pour le réglage en hauteur du dispositif porteur (19), guidage qui coopère avec un organe coulissant (27) du dispositif porteur (19).

5. Dispositif selon la revendication 4, **caractérisé en ce que** la colonne destinée à réaliser le guidage linéaire est conçue à la manière d'un tube (11), en particulier avec une section intérieure circulaire ou rectangulaire.

6. Dispositif selon la revendication 4 ou 5, **caractérisé en ce que** l'organe coulissant comprend au moins deux éléments coulissants (27) écartés l'un de l'autre en direction d'un axe longitudinal de la colonne (11), réalisés en particulier sous forme de patins à gorge.

7. Dispositif selon la revendication 6, **caractérisé en ce que** les éléments coulissants (27) sont réalisés sous forme sphérique pour leur montage dans un tube (11) qui présente en particulier une section intérieure circulaire.

8. Dispositif selon la revendication 6, **caractérisé en ce que** les éléments coulissants (27) sont réalisés sous forme cylindrique pour leur montage dans un tube (11) qui présente en particulier une section intérieure rectangulaire.

9. Dispositif selon l'une des revendications précédentes, **caractérisé en ce qu'**un corps de base (35) du dispositif porteur (19) est capable de pivoter par rapport à la colonne (11) autour d'un premier axe de pivotement (41) orienté en direction médiale-latérale, pour corriger l'inclinaison de la surface de guidage du bloc de coupe par rapport à l'axe sagittal.

10. Dispositif selon la revendication 9, **caractérisé en ce qu'**au moins un élément coulissant (27b) est réalisé pour le réglage en hauteur du dispositif porteur (19) et simultanément pour le pivotement du corps de base (35) du dispositif porteur (19).

11. Dispositif selon la revendication 9 ou 10, **caractérisé en** se que pour le pivotement du corps de base (35), la distance d'au moins un élément coulissant (27b) par rapport au corps de base (35) peut être variée.

12. Dispositif selon la revendication 11, **caractérisé en ce que** l'élément coulissant (27b) et le corps de base (35) sont reliés l'un à l'autre au moyen d'un vissage de positionnement (37).

13. Dispositif selon l'une des revendications 6 à 12, **caractérisé en ce que** les éléments coulissants (27) sont respectivement reliés à un corps de base (35) via un élément de liaison (33) étiré, en particulier en longueur, et au moins un élément de liaison (33b) est de préférence pourvu d'un pas de vis et d'un vissage de positionnement (37).

14. Dispositif selon l'une des revendications précédentes, **caractérisé en ce que** la colonne (11) est au moins localement fendue, le bras porteur (21) étant passé à travers la fente (29) réalisée dans la colonne (11), en particulier entre deux éléments coulissants (27).

15. Dispositif selon la revendication 14, **caractérisé en ce qu'**au moins un élément de blocage (43) du dispositif porteur (19) est en engagement avec la fente (29) pour le blocage antirotation du bras porteur (21) à l'encontre d'une rotation autour d'un axe longitudinal de la colonne (11).

16. Dispositif selon la revendication 15, **caractérisé en ce que** l'élément de blocage (43) est monté sur un corps de base (35) du dispositif porteur (19).

17. Dispositif selon la revendication 15 ou 16, **caractérisé en ce que** l'élément de blocage (43) est monté sur un élément coulissant (27) du dispositif porteur (19).

18. Dispositif selon l'une des revendications précédentes, **caractérisé qu'**en ce qu'un élément de monture (45) du dispositif porteur (19) est capable de pivoter autour d'un second axe de pivotement (47) orienté en direction antérieure/postérieure pour corriger l'inclinaison de la surface de guidage du bloc de coupe par rapport à l'axe transversal.

19. Dispositif selon la revendication 18, **caractérisé en ce que** l'élément de monture (45) est réalisé sous forme d'un élément d'entraînement pour le bras porteur (21).

20. Dispositif selon la revendication 18 ou 19, **caractérisé en ce que**, pour le pivotement de l'élément de monture (45), il est prévu une tige filetée (49) réglable en direction médiale-latérale, et dans laquelle s'engage l'élément de monture (45).

21. Dispositif selon l'une des revendications précédentes, **caractérisé en ce que** l'angle d'un mouvement de pivotement autour d'un premier et/ou d'un second axe (41, 47) respectivement est lisible sur une échelle (42).
